# EUROPEAN PATENT SPECIFICATION

(11) **EP 1 515 749 B1**
(45) Date of publication and mention of the grant of the patent: **15.08.2012**
(21) Application number: 03759875.2
(22) Date of filing: 12.06.2003
(51) Int. Cl.: A61K 39/395, A61K 38/26, A61P 3/10

(54) **COMBINED USE OF A MODULATOR OF CD3 AND A GLP-1 COMPOUND**
KOMBINIERTE VERWENDUNG EINES MODULATORS VON CD3 UND EINE GLP-1 VERBINDUNG
UTILISATION COMBINEE D'UN MODULATEUR DE CD3 ET D'UN COMPOSE A BASE DE GLP-1

(30) Priority: 14.06.2002 DK 200200909
(43) Date of publication of application: 23.03.2005
(73) Proprietor: NOVO NORDISK A/S, 2880 Bagsværd (DK)
(72) Inventor: MICHELSEN, Birgitte, Koch, DK-2800 Lyngby (DK); STURIS, Jeppe, Dk-3500 Vaerlose (DK)
(86) International application number: PCT/DK2003/000387
(87) International publication number: WO 2003/105897

(56) References cited:
- WO-A-01/35988
- WO-A-95/31214
- WO-A-99/43708
- US-A- 5 834 597
- KEVAN C. HEROLD ET AL: "Anti-CD3 Monoclonal antibody in new-onset type 1 diabetes mellitus " N ENGL J MED, vol. 346, no. 22, 30 May 2002 (2002-05-30), pages 1692-1698, XP002255982
- L. CHATENOUD: "Restoration of self-tolerance is a feasible approach to control ongoing beta-cell specific autoreactivity: its relevance for treatment in established diabetes and islet transplantation" DIABETOLOGIA, vol. 44, 2001, pages 521-536, XP002255983
- THOMAS HAAK: "New developments in the treatment of type 1 diabetes mellitus " EXP CLIN ENDOCRINOL DIABETES, vol. 107, no. 3, 1999, pages S108-S113, XP002255984
- SHERRY, N.A. ET AL ENDOCRINOLOGY no. 148, 2007, pages 5136 - 5144
- VERNERIS, M.R. ET A BIOL. BLOOD no. 7, 2001, pages 532 - 542
- CLARK, D.M. AND A. W. BOYLSTON IMMUNOL. METHODS no. 106, 1998, pages 127 - 133
- XU, D. ET AL CELLULAR IMMUNOL. no. 200, 2000, pages 16 - 26
- KNUDSEN, L.B. ET AL J. MED. CHEM. no. 43, 2000, pages 1664 - 1669
- AGERSO, H. ET AL DIABETOLOGIA no. 45, 2002, pages 195 - 202

## Description

### FIELD OF THE INVENTION

The present invention relates to methods and uses for the prevention and intervention of Type 1 diabetes and intervention of Latent Autoimmune Diabetes in the Adult (LADA). More specifically, the methods and uses of the invention pertain to administration of a modulator of CD3 in combination with administration of a GLP-1 compound.

### BACKGROUND OF THE INVENTION

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterised as Type 1 diabetes and insufficient utilization of insulin is Type 2 diabetes.

Type 1 diabetes mellitus is caused by an autoimmune destruction of the pancreatic beta cells. Likewise, in Latent Autoimmune Diabetes in the Adult (LADA), autoimmunity accelerates the disease process in patients initially diagnosed with Type 2 diabetes, leading to rapid progression to insulin requirement in these patients. T cells play an important role in this process by mediating the autoimmune destruction. It has therefore been hypothesized that it may be possible to influence the development of Type 1 diabetes mellitus as well as the disease progression in LADA patients by regulation of T cells or of the immune system's response to T cells.

CD3 is expressed in T cells. It has been recently demonstrated in humans that short term treatment of new onset Type 1 diabetic patients with an antibody against CD3 is able to attenuate the further destruction of beta-cells, thereby facilitating improved glycemic control of the patients. Ultimately, this gives the patients a better prognosis with respect to the development of diabetic late complications.

Chatenoud, L. et al. (2001) Diabetologia 44521 -536 relates to immunointervention in type 1 diabetes aimed at restoring self-tolerance to beta cells using, e.g., anti-CD3. Haak T. et al. (1999) Exp. Clin. Endocrinol. Diabetes 107 (Suppl. 3) S108-S113 relates to treatment of type 1 diabetes and describes that GLP-1 lowers blood glucose in both type 1 and type 2 diabetic patients. WO 95/31214 relates *inter alia* to a method of treating insulin- requiring diabetes in a mammal comprising administering to the mammal in a suitable regimen an effective amount of insulin and an effective amount of a peptide comprising a peptide selected from the group consisting of (a) glucagon-like peptide 1(7-37); (b) glucagon-like peptide 1(7-36)amide; and (c) an effective fragment or analogue of (a) or (b). WO 01/35988 relates *inter alia* to the use of a GLP-1 agonist for the preparation of a medicament for treatment of beta cell degeneration. Knudsen, LB. et al (2000) J. Med. Chem. 43:1664-1669 relates to potent derivatives of GLP-1 suitable for once daily administration. Agersø, H. et al (February 2002) Diabetologia 45:195-202 relates to pharmacokinetics, pharmacodynamics, safety and tolerability of the new long-acting GLP-1 derivative NN2211.

The present invention concerns the combined treatment with at least one compound that regulates CD3 and at least one GLP-1 compound. The treatment can either be prophylactic, i.e. given to a subject at high risk for the development of Type 1 diabetes, or as an intervention in the disease process at the time it is clinically diagnosed. By this combined treatment, it is possible to avoid the further destruction of beta cells. The treatment can furthermore lead to an increase of beta-cell function as measured by C-peptide after treatment has been discontinued. The effect will be sustained over several years, thereby having a major beneficial impact on the severity of the disease and its complications. Patients will receive state-of-the art therapy with insulin and/or insulin analogs simultaneously during the treatment period in order to provide glycemic control.

In accordance with the present invention, a pharmaceutical combination is provided for use in the prevention and intervention of Type 1 diabetes and LADA, which combination comprises a modulator of CD3 and a GLP-1 compound.

### SUMMARY OF THE INVENTION

One object of the present invention is to provide methods, which can effectively be used in the in the prevention and intervention of Type 1 diabetes and intervention of LADA.

The present invention includes the use of a modulator of CD3 and a GLP-1 compound for the preparation of one ore more medicaments for the prevention and intervention of Type 1 diabetes and LADA in a patient in need thereof.

In one embodiment of the invention, the modulator of CD3 is selected from antibody reactive with CD3 or F(ab')2 fragment of said antibody.

In another embodiment of the invention the modulator of CD3 is OKT3, hOKT3γ1(Ala-Ala), or 145 2C11.

In another embodiment of the invention the modulator of CD3 is anti-CD3 mAb hOKT3γ1 (Ala-Ala).

In aanother embodiment of the invention the modulator of CD3 is anti-CD3 mAb 145 2C11 or F(ab')2 fragment thereof.

In another embodiment of the invention the GLP-1 compound is selected from GLP-1 (7-36)-amide, GLP-1 (7-37), an analogue thereof and a derivative of any of the foregoing.

In another embodiment of the invention the GLP-1 compound is a stable GLP-1 analog/derivative.

In another embodiment of the invention the GLP-1 compound is Arg³⁴, Lys²⁶(N^{ε}-(γ-Glu(N^{α}-hexadecanoyl)))-GLP-1(7-37).

In another embodiment of the invention the modulator of CD3 and the GLP-1 compound are administered in suboptimal dosages.

In yet another embodiment of the invention the modulator of CD3 and the GLP-1 compound are administered in amounts and for a sufficient time to produce a synergistic effect.

### DEFINITIONS

Co-administration: In the context of the present application, co-administration of two compounds is defined as administration of the two compounds to the patient within one year, including separate administration of two medicaments each containing one of the compounds as well as simultaneous administration whether or not the two compounds are combined in one formulation or whether they are in two separate formulations.

Effective dosage: An effective dosage is a dosage which is sufficient in order for the treatment of the patient to be effective compared with no treatment.

Medicament: Pharmaceutical composition suitable for administration of the pharmaceutically active compound to a patient.

Suboptimal dosage: A suboptimal dosage of a pharmaceutically active compound is a dosage which is below the optimal dosage for that compound when used in single-compound therapy.

Additive effect: An additive effect of two compounds is an effect equal to the sum of the effects of the two individual compounds.

Synergistic effect: A synergistic effect of two compounds is in terms of statistical analysis an effect which is greater than the additive effect which results from the sum of the effects of the two individual compounds.

Favourable effect: A favourable effect of two compounds is in terms of statistical analysis an effect which is greater than the effect of either of the two compounds alone.

Prevention and intervention of Type 1 diabetes and LADA (Latent Autoimmune Diabetes in the Adult): In this application prevention is defined as the management and care of an individual at risk of developing Type 1 diabetes or LADA prior to the clinical onset of the disease. Intervention is defined as the management and care of a Type 1 or LADA diabetes patient at diagnosis or later. The purpose of prevention and intervention is to combat the disease, condition, or disorder and includes the administration of the active compounds to prevent or delay the onset of the symptoms or complications, or alleviating the symptoms or complications, or eliminating the disease, condition, or disorder.

Modulator of CD3: In this application a modulator of CD3 is defined as a compound that interacts with CD3 and modulates the effects of CD3, such as an antibody reactive with CD3.

GLP-1 compound : In this application a GLP-1 compound refers to GLP-1(1-37), exendin-4(1-39), insulinotropic fragments thereof, insulinotropic analogs thereof and insulinotropic derivatives thereof. Insulinotropic fragments of GLP-1(1-37) are insulinotropic peptides for which the entire sequence can be found in the sequence of GLP-1(1-37) and where at least one terminal amino acid has been deleted. Examples of insulinotropic fragments of GLP-1(1-37) are GLP-1(7-37) wherein the amino acid residues in positions 1-6 of GLP-1(1-37) have been deleted, and GLP-1 (7-36) where the amino acid residues in position 1-6 and 37 of GLP-1(1-37) have been deleted. Examples of insulinotropic fragments of exendin-4(1-39) are exendin-4(1-38) and exendin-4(1-31). The insulinotropic property of a compound may be determined by in vivo or in vitro assays well known in the art. For instance, the compound may be administered to an animal and monitoring the insulin concentration over time. Insulinotropic analogs of GLP-1 (1-37) and exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound . Examples of insulinotropic analogs of GLP-1(1-37) is e.g. Met⁸-GLP-1(7-37) wherein the alanine in position 8 has been replaced by methionine and the amino acid residues in position 1 to 6 have been deleted, and Arg³⁴-GLP-1(7-37) wherein the valine in position 34 has been replaced with arginine and the amino acid residues in position 1 to 6 have been deleted. An example of an insulinotropic analog of exendin-4(1-39) is Ser²Asp³-exendin-4(1-39) wherein the amino acid residues in position 2 and 3 have been replaced with serine and aspartic acid, respectively (this particular analog also being known in the art as exendin-3). Insulinotropic derivatives of GLP-1(1-37), exendin-4(1-39) and analogs thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups and lipophilic substituents. Examples of insulinotropic derivatives of GLP-1(1-37), exendin-4(1-39) and analogs thereof are GLP-1(7-36)-amide, Arg³⁴, Lys²⁶(N^{ε}-(γ-Glu(N^{α}-hexadecanoyl)))-GLP-1(7-37) and Tyr³¹-exendin-4(1-31)-amide. Further examples of GLP-1(1-37), exendin-4(1-39), insulinotropic fragments thereof, insulinotropic analogs thereof and insulinotropic derivatives thereof are described in WO 98/08871, WO 99/43706, US 5424286 and WO 00/09666.

"Stable GLP-1 analog/derivative". In this application a stable GLP-1 analog/derivative refers to a GLP-1(1-37) analog or derivative thereof which exhibits an in vivo plasma elimination half-life of at least 10 hours in man, as determined by the method described below. Examples of stable derivatives of GLP-1 analogs can be found in WO 98/08871 and WO 99/43706. The method for determination of plasma elimination half-life of a compound in man is : The compound is dissolved in an isotonic buffer, pH 7.4, PBS or any other suitable buffer. The dose is injected peripherally, preferably in the abdominal or upper thigh. Blood samples for determination of active compound are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active compound is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.

"Stable exendin-4 analog/derivative". As used herein the term "stable exendin-4 analog/derivative" refers to a exendin-4(1-39) analog or derivative thereof which exhibits an in vivo plasma elimination half-life of at least 10 hours in man, as determined by the method described above.

### DETAILED DESCRIPTION OF THE INVENTION

It has been discovered that in the prevention and intervention of Type 1 diabetes and LADA, the combined treatment with a modulator of CD3 and a GLP-1 compound avoids further destruction of beta cells. It has also been discovered that the combined treatment leads to an increase of beta cell function. This increase in beta-cell function may be sustained over several years and gives the patient an improved glycemic control and improved prognosis with respect to microvascular and macrovascular complications.

A synergistic effect of two compounds permits the dosages of these compounds in the combined treatment to be below the optimal dosages of the individual compounds in single-compound treatment Thus, these suboptimal dosages of the individual compounds reduce side effects since lower dosages are needed for the same therapeutic effect in the combined treatment.

Furthermore, a synergistic effect of the two compounds permits the efficacy of the co-administration of the two compounds to be significantly greater than the sum of the efficacy of each individual compound.

In another aspect the present invention relates to use of a modulator of CD3 and a GLP-1 compound for the preparation of one or more medicaments for the prevention and intervention of Type 1 diabetes and LADA in a patient in need thereof.

The methods comprise administration of an effective amount of a modulator of CD3 and administration of an effective amount of a GLP-1 compound. The two compounds may be co-administered or they may be administered separately as two medicaments. Furthermore, the first compound may be administered in a regimen, which additionally comprises treatment with the second compound.

In one embodiment of the invention, the modulator of CD3 is a CD3 antibody or F(ab')2 fragment thereof or other CD3 binding compound with same activity.

In another embodiment of the invention, the modulator of CD3 is anti-CD3 monoclonal antibody OKT3 or F(ab')2 fragment thereof.

In another embodiment of the invention, the modulator of CD3 is hOKT3y1 (Ala-Ala) or F(ab')2 fragment thereof.

In another embodiment of the invention the modulator of CD3 is CD3 mAb 145 2C11 or F(ab')2 fragment thereof.

In another embodiment the GLP-1 compound is GLP-1(7-37). GLP-1(7-36) amide, or an analog thereof or a derivative of any of the foregoing.. Such GLP-1 compounds include, but are not limited to, Arg²⁶-GLP-1(7-37); Arg³⁴-GLP-1(7-37); Lys³⁶-GLP-1(7-37): Arg^{26,34}Lys³⁶-GLP-1(7-37); Arg^{26,34}-GLP-1(7-37); Arg^{26,34}Lys⁴⁰-GLP-1(7-37)LP-1(7-37): Arg²⁶Lys³⁶-GLP-1(7-37); Arg³⁴Lys³⁶- GLP-1(7-3T); Val⁸Arg²²-GLP-1(7-37); Met⁸Arg²²-GLP-1(7-37);Gly⁸His²²-GLP-1(7-37); Val⁸His²²-GLP-1(7-37); Met⁸His²²-GLP-1(7-37);His³⁷-GLP-1(7-37); Gly⁸-GLP-1(7-37); Val⁸-GLP-1(7-37); Met⁸-GLP-1(7-37);Gly⁸Asp²²-GLP-1(7-37); Val⁸Asp²²-GLP-1(7-37); Met⁸Asp²²-GLP-1(7-37);GIly⁸Glu²²-1(7-37); Val²²Glu²²-GLP-1(7-37); Met⁸Glu²²-GLP-1(7-37); Gly⁸Lysn²²-GLP-1(7-37); Val⁸Lys²²-GLP-1(7-37); Met⁸Lys²²-GLP-1(7-37); Gly⁸Arg²²-GLP-1(7-37); Val⁸Lys²²His²²-GLP-1(7-37); Gly⁸Glu²²His³⁷-GLP-1(737); Val⁸Glu²²His³⁷-GLP. 1(7-37); Met⁸Glu²²His³⁷-GLP-1(7-37);Gly⁸Lys²² His³⁷-GLP-1(7-37); Met⁸Lys²²His-GLP-1(7-37);Gly⁸Arg²²His³⁷-GLP-1(7-37); Val⁸Arg²²His³⁷-GLP-1(7-37): Met⁸Arg²²His³⁷-GLP-1(7-37); Gly⁸His²²His³⁷-GLP-1(7-37); Val⁸His²²His³⁷-GLP-1(7-37); Met⁸His²²His³⁷-GLP-1(7-37); Gly⁸His³⁷-GLP-1 (7-37); Val⁸His³⁷-GLP-1 (7-37); Met⁸His³⁷-GLP-1 (7-37);Gly⁸ Asp²² His³⁷-GLP-1(7-37); Val⁸Asp²²His³⁷-GLP-1(7-37); Met⁸Asp²²His³⁷-GLP-1 (7-37); Arg²⁶-GLP-1 (7-36)-amide; Arg³⁴-GLP-1 (7-36)-amide; Lys³⁶-GLP-1(7-36)-amide; Arg^{26,34}Lys³⁶-GLP-1 (7-36)-amide; Arg^{28,34}-GLP-1(7-36)-amide; Arg^{26.34}Lys⁴⁰-GLP-1(7-36)-amide; Arg²⁶Lys³⁸ -GLP-1 (7-36)-amide; Arg³⁴Lys36 -GlP-1 (7-36)-amide; Gly⁸-GLP-1 (7-36)-amide; Val⁸-GLP-1(7-36)-amide; Met⁸-GLP-1(7-36)-amide;Gly⁸Asp²²-GLP-1(7-36)-amide; Gly⁸Glu²²His³⁷-GLP-1(7-36)-amide; Val⁸Asp²²-GLP-1(7-36)-amide; Met⁸Asp²²-GLP-1(7-36)-amide;Gly⁸Glu²²-GLP-1 (7-36)-amide; Val⁸Glu²²-GLP-1 (7-36)-amide; Met⁸Glu²²-GLP-1(7-36)-amide; Gly⁸Lys²²-GLP-1(7-36)-amide; Val⁸Lys²²-GLP-1(7-36)-amide; Met⁸Lys²²-GLP-1(7-36)-amide; Gly⁸His²²His³⁷-GLP-1(7-36)-amide; Gly⁸Arg²²-GLP-1(7-36)-amide; Val⁸Arg²²-GLP-1(7-36)-amide; Met⁸Arg²²-GLP-1(7-36) amide;Gly⁸His²²-GLP-1(7-36)amide; Val⁸His²²-GLP-1(7-36)-amide; Met⁸His²²-GLP-1(7-36)-amide;His³⁷-GLP-1(7-36)-amide; Val⁸Arg²²His³⁷-GLP-1(7-36)-amide; Met⁸Arg²²His³⁷-GLP-1(7-36)-amide; Gly⁸His³⁷-GLP-1(7-36)-amide; Val⁸His³⁷-GLP-1(7-36)-amide; Met⁸His³⁷-GLP-1(7-36)-amide;Gly⁸Asp²² His³⁷-GLP-1(7-36)-amide; Val⁸Asp²²His³⁷-GLP-1(7-36)-amide; Met⁸ Asp²²His³⁷-GLP-1 (7-36)-amide; Val⁸Glu²²His³⁷-GLP-1 (7-36)-amide; Met⁸Glu²²His³⁷ -GLP-1(7-36)-amide;Gly⁸Lys²² His³⁷-GLP-1 (7-36)-amide; Val⁸Lys²²His³⁷-GLP-1(7-36)-amide; Mel⁸Lys²²His³⁷-GLP-1(7-36)-amide;Gly⁸ Arg²²His³⁷-GLP-1(7-36)-amide; Val⁸His²² His³⁷ -GLP-1(7-36)-amide; Met⁸His²²His³⁷-GLP-1 (7-36)-amide; and derivatives thereof. In another embodiment of the invention the GLP-1 compound is is selected from the group consisting of Gly⁸-GLP-1(7-36)-amide, Gly⁸-GLP-1 (7-37), Val⁸-GLP-1 (7-36)-amide, Val⁸-GLP-1 (7-37), Val⁸Asp²²-GLP-1(7-36)-amide, Val⁸Asp²²-GLP-1 (7-37) , Val⁸Glu²²-GLP-1(7-36)-amide , Val⁸Glu²²-GLP-1 (7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1(7-37), Val⁸His²²-GLP-1(7-36)-amide, Val⁸His²²-GLP-1 (7-37), analogues thereof and derivatives thereof.

In another embodiment the GLP-1 compound is exendin-4.

In another embodiment the GLP-1 compound is a stable GLP-1 analog/derivative.

In another embodiment the GLP-1 compound is a derivative of GLP-1 (7-36)-amide or GLP-1(7-37) which comprises a lipophilic substituent.

In another embodiment the GLP-1 compound is is Arg³⁴, Lys²⁶(N^{ε}-(γ-Glu(N^{α}-hexadecanoyl)))-GLP-1 (7-37).

GLP-1 analogues and derivatives which can be used according to the present invention includes those referred to in WO 99/43705 (Novo Nordisk A/S), WO 99/43706 (Novo Nordisk A/S), WO 99/43707 (Novo Nordisk A/S), WO 98/08871 (Novo Nordisk A/S), WO 99/43708 (Novo Nordisk A/S), WO 99/43341 (Novo Nordisk A/S), WO 87/06941 (The General Hospital Corporation), WO 90/11296 (The General Hospital Corporation), WO 91/11457 (Buckley et al.), WO 98/43658 (Eli Lilly & Co.), EP 0708179-A2 (Eli Lilly & Co.), EP 0699686-A2 (Eli Lilly & Co.), WO 01/98331 (Eli Lilly & Co).

In another embodiment of the invention the GLP-1 compound is selected from exendin as well as analogs, derivatives, and fragments thereof, e.g. exendin-3 and exendin-4. Examples of exendins as well as analogs, derivatives, and fragments thereof to be included within the present invention are those disclosed in WO 9746584, US 5424286 and WO 01/04156. US 5424286 describes a method for stimulating insulin release with an exendin polypeptide. The exendin polypeptides disclosed include HGEGTFTSDLSKOMEEEAVRLFIEWLKNGGX; wherein X = P or Y, and HX1X2GTFITSDLSKQMEEEAVRLFIEWLKNGGPSSGAPPPS; wherein X1X2 = SD (exendin-3) or GE (exendin-4)). WO 9746584 describes truncated versions of exendin peptide(s). The disclosed peptides increase secretion and biosynthesis of insulin, but reduce those of glucagon. WO 01/04156 describes exendin-4 analogs and derivatives as well as the preparation of these molecules.

In another embodiment of the invention the GLP-1 compound is a stable exendin-4 analog/derivative.

GLP-1 compounds can be produced by appropriate derivatization of an appropriate peptide backbone which has been produced by recombinant DNA technology or by peptide synthesis (e.g. Merrifield-type solid phase synthesis) as known in the art of peptide synthesis and peptide chemistry.

Modulators of CD3 such as anti-CD3 mAb may be produced by mammalian cell culture known in the art of mAb production.

In another embodiment of the invention the modulator of CD3 and the GLP-1 compound are co-administered to the patient. The two compounds may be administered as separately formulated compounds or they may be administered as one formulation comprising both compounds.

In a further embodiment, the modulator of CD3 is administered in a regimen, which additionally comprises administration of the GLP-1 compound.

In yet another embodiment, the modulator of CD3 and the GLP-1 compound are administered in suboptimal dosages, i.e. dosages lower than the optimal dosages for single compound therapy.

In a further embodiment the modulator of CD3 and the GLP-1 compound are administered in sufficient amount and for a sufficient time to produce a favourable effect.

In a further embodiment the modulator of CD3 and the GLP-1 compound are administered in sufficient amount and for a sufficient time to produce an additive effect.

In a further embodiment the modulator of CD3 and the GLP-1 compound are administered in sufficient amount and for a sufficient time to produce a synergistic effect.

The subject or patient is preferably a mammal, more preferably a human.

The route of administration may be any route, which effectively transports the active compound to the appropriate or desired site of action, such as oral, nasal, buccal, pulmonal, transdermal or parenteral.

Pharmaceutical compositions (or medicaments) containing the modulator of CD3, such as OKT3, hOKT3γ1(Ala-Ala), or 145 2C11 may be administered by suitable dosage forms such as parenteral.

The route of administration of GLP-1 compounds and of modulators of CD3 may be any route, which effectively transports the active compound to the appropriate or desired site of action, such as oral, nasal, buccal, pulmonal, transdermal or parenteral.

Medicaments or pharmaceutical compositions containing a GLP-1 compound or modulators of CD3, such as Arg³⁴, Lys²⁶(N^{ε}-(γ-Glu(Nα-hexadecanoyl)))-GLP-1 (7-37), may be administered parenterally to a patient in need thereof. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of a GLP-1 compound in the form of a nasal or pulmonal spray. As a still further option, the GLP-1 compound can also be administered transdermally, *e.g.* from a patch, optionally a iontophoretic patch, or transmucosally, *e.g*. bucally. The above-mentioned possible ways to administer GLP-1 compounds are not considered as limiting the scope of the invention.

Pharmaceutical compositions containing GLP-1 compounds and/or modulators of CD3, such as Arg³⁴ , Lys²⁶(N^{ε}-(γ-Glu(N^{α}-hexadecanoyl)))-GLP-1(7-37) and/or anti-CD3 mAb hOKT3γ1(Ala-Ala), may be prepared by conventional techniques, *e.g*. as described in Remington's Pharmaceutical Sciences, 1985 or in Remington: The Science and Practice of Pharmacy, 19th edition, 1995.

Thus, the injectable compositions of GLP-1 compounds and of modulators of CD3 can be prepared using the conventional techniques of the pharmaceutical industry which involves dissolving and mixing the ingredients as appropriate to give the desired end product.

According to one procedure, e.g. Arg^{3A}, Lys²⁶(N^{ε}-(γ-Glu(N^{α}-hexadecanoyl)))-GLP-1 (7-37) is dissolved in an amount of water which is somewhat less than the final volume of the composition to be prepared. An isotonicity agent, a preservative and a buffer are added as required and the pH value of the solution is adjusted - if necessary - using an acid, *e.g*. hydrochloric acid, or a base, *e.g*. aqueous sodium hydroxide as needed. Finally, the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

Further to the above-mentioned components, solutions containing a GLP-1 compound may also contain a surfactant in order to improve the solubility and/or the stability of the peptide.

According to one embodiment of the present invention, the GLP-1 compound and modulators of CD3 is provided in the form of a composition suitable for administration by injection. Such a composition can either be an injectable solution ready for use or it can be an amount of a solid composition, *e.g*. a lyophilised product, which has to be dissolved in a solvent before it can be injected. The injectable solution preferably contains not less than about 0.1 mg/ml GLP-1 compound, typically from 0.1 mg/ml to 10 mg/ml GLP-1 compound, such as from 1 mg/ml to 5 mg/ml of GLP-1 compound.

GLP-1 compounds such as Arg³⁴, Lys²⁶(N^{ε}-(γ-Glu(N^{α}-hexadecanoyl)))-GLP-1(7-37) can be used in the treatment of Type 1 diabetes and LADA patients and/or potential Type 1 diabetes patients and potential LADA patients. The optimal dose level of GLP-1 compound and of modulator of CD3 for any patient (effective amount) will depend on the history and state of that particular patient to be treated. The person skilled in the art, e.g. a physician, will know how to determine the optimal dose level in order to control the blood glucose level within intervals mentioned herein.

The GLP-1 compounds are effective over a wide dosage range. For example, in the treatment of adult humans, dosages from 0.01 µg/kg/day to 100 µg/kg/day, preferably from 0.1 µg/kg/day to 30 µg/kg/day may be used. A most preferable dosage is from 2 µg/kg/day to 20 µg/kg/day. The exact dosage will depend upon the mode of administration, on the therapy desired, the administration form, the subject to be treated and the body weight of the subject to be treated.

The treatment with the modulator of CD3 may be repeated at intervals ranging from every 3 months to every 10 years.

The treatment with the beta-cell resting compound may be repeated at intervals ranging from every 3 months to every 10 years.

The treatment with the beta-cell resting compound may be daily for the lifetime of the patient.

Irrespective of the dosage forms for the modulator of CD3 and for the GLP-1 compound, they may advantageously be supplied as a kit for the prevention and intervention of Type 1 diabetes. The kit may contain a single dosage form or it may contain two dosage forms, i.e. one for each compound to be administered. In one embodiment the kit contains a fixed ratio dosage of the GLP-1 compound and modulator of CD3.

The combined treatment with a modulator of CD3 and a GLP-1 compound may also be combined with a third or more further pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Most importantly, when the treatment is used in already diagnosed Type 1 or LADA diabetic patients, co-therapy with insulin, insulin analogues or oral antidiabetic agents will be common. Examples of these pharmacologically active substances are : Insulin, GLP-1 agonists, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), compounds lowering food intake, RXR agonists and agents acting on the ATP-dependent potassium channel of the β-cell; Cholestyramine, colestipol, clofibrate, gemfibrozil, lovastatin, pravastatin, simvastatin, probucol, dextrothyroxine; β-blockers such as alprenolol, atenolol, timolol, pindolol, propranolol and metoprolol, ACE (angiotensin converting enzyme) inhibitors such as benazeprit, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4)
agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists.

### EXAMPLES

### Example 1

The synergistic effects of the combined use of a modulator of CD3 and a GLP-1 compound can be measured as follows:

### Eighty type 1 diabetic patients at diagnosis.

### Study design and treatment protocols:

Upon enrollment and following informed consent, patients are be-randomized into one of four groups: one receiving anti-CD3 and placebo for a GLP-1 compound, one receiving placebo for anti-CD3 and a GLP-1 compound, one receiving both anti-CD3 and a GLP-1 compound and one receiving placebo for both anti-CD3 and a GLP-1 compound. Anti-CD3 treatment or placebo is administered at a schedule as described by Herold et. al. N Engl J Med 346:1692-98, 2002. Starting the same day as the anti-CD3 treatment, the GLP-1 compound is administered 4 times daily for a period of 3 months. This 3 month period is referred to as the treatment period. Patients receive state-of-the art therapy with insulin and/or insulin analogs simultaneously during the treatment period in order to provide glycemic control.

### Endpoints:

The primary endpoint is area under the curve for insulin secretion rates quantified by deconvolution of C-peptide concentrations for the meal test performed after the three month treatment period. Secondary endpoints is fasting C-peptide, insulin secretion rates after the oral glucose tolerance test, use of exogenous insulin, and HbA1c. The statistical analysis is based on baseline subtracted data.

### Baseline assessment and data collection

At baseline, subjects or patients have fasting C-peptide, an oral glucose tolerance test and a meal tolerance test performed. Their islet cell antibodies are assessed. The following period, treatment occur. They receive intensive insulin therapy in order to provide glycemic control. They are also receiving (and-CD3 or placebo) and (the GLP-1 compound or placebo). Between one and seven days after the treatment period has ended and every three months thereafter for an indefinite period, HbA1 c fasting C-peptide, oral glucose tolerance tests and meal tests are repeated.

### Statistical analysis

The statistical analysis shows a synergistic effect on the primary endpoint, i.e. the effect of combining anti-CD3 and the GLP-1 compound is greater than the additive effect of either treatment regimen alone. If the effect of administering placebo for anti-CD3 and placebo for the GLP-1 compound is designated A, the effect of administering anti-CD3 and placebo for the GLP-1 compound is designated B, the effect of administering placebo for anti-CD3 and the GLP-1 compound is designated C and the effect of administering anti-CD3 and the GLP-1 compound is designated D, then the statistical analysis shows that D-A is greater than (B-A)+(C-A) with statistical significance at the 0.05 level. The statistical test used is a two-way analysis of variance with anti-CD3 and the GLP-1 compound as the two factors. The interaction term is used to ascertain the presence of synergy.

## Claims

1. A modulator of CD3 and a glucagon-like peptide-1 (GLP-1) compound for use in the prevention and intervention of Type 1 diabetes and Latent Autoimmune Diabetes in the Adult (LADA) for administration to a patient in need thereof, wherein said modulator is a CD3 antibody or F(ab')2 fragment thereof.

2. The modulator of CD3 and GLP-1 compound for use according to claim 1, wherein the modulator of CD3 is selected from the group consisting of anti-CD3 mAb OKT3 or F(ab')2 fragment thereof, anti-CD3 mAb hOKT3γ1(Ala-Ala) or F(ab')2 fragment thereof or anti-CD3 mAb 145 2C11 or F(ab')2 fragment thereof.

3. The modulator of CD3 and GLP-1 compound for use according to any one of the preceding claims wherein the GLP-1 compound is selected from the group consisting of GLP-1 (7-36)-amide, GLP-1 (7-37), an analogue thereof and a derivative of any of the foregoing.

4. The modulator of CD3 and GLP-1 compound for use according to any one of claims 1-3, wherein the GLP-1 compound is a derivative of GLP-1 (7-36)-amide, GLP-1 (7-37) or an analog of any of the foregoing which comprises a lipophilic substituent.

5. The modulator of CD3 and GLP-1 compound for use according to any one of claims 1-4, wherein the GLP-1 compound is Arg³⁴, Lys²⁶(N^{ε}-(γ-Glu(N^{α}-hexadecanoyl)))-GLP-1(7-37).

6. The modulator of CD3 and GLP-1 compound for use according to any one of claims 1-4, wherein the GLP-1 compound is selected from the group consisting of Gly⁸-GLP-1(7-36}-amide, Gly⁸ -GLP-1(7-37), Val⁸-GLP-1 (7-36)-amide, Val⁸-GLP-1 (7-37), Val⁸Asp²²-GLP-1 (7-36)-amide, Val⁸Asp²²-GLP-1(7-37), Val⁸Glu²²-GLP-1(7-36)-amide, Val⁸Glu²²-GLP-1 (7-37), Val⁸Lys²²-GLP-1 (7-36)-amide, Val⁸Lys²²-GLP-1(7-37), Val⁸Arg²²-GLP-1(7-36)-amide. Val⁸Arg²²-GLP-1 (7-37), Val⁸His²²-GLP-1(7-36)-amide, Val⁸His²²-GLP-1 (7-37), analogues thereof and derivatives thereof.

7. The modulator of CD3 and GLP-1 compound for use according to any one of claims 1-2, wherein the GLP-1 compound is a stable GLP-1 analog/derivative.

8. The modulator of CD3 and GLP-1 compound for use according to any one of claims 1-2, wherein the GLP-1 compound is exendin-4 or an analogue thereof or a derivative of any of the foregoing.

9. The modulator of CD3 and GLP-1 compound for use according to any one of claims 1-2, wherein the GLP-1 compound is a stable exendin-4 analog/derivative.

10. The modulator of CD3 and GLP-1 compound for use according to any one of the preceding claims, wherein the GLP-1 compound is to be administered parenterally or by injection.

11. The modulator of CD3 and GLP-1 compound for use according to claim 10, wherein the dosage of GLP-1 compound is from about 0.5 µg/kg/day to about 20 µg/kg/day, such as from about 0.1 µg/kg/day to about 2 µg/kg/day.

12. The modulator of CD3 and GLP-1 compound for use according to any one of the preceding claims, wherein the modulator of CD3 is administered in a regimen which additionally comprises administration of a GLP-1 compound.

13. The modulator of CD3 and GLP-1 compound for use according to any one of the preceding claims, wherein the modulator of CD3 and the GLP-1 compound are co-administered.

14. The modulator of CD3 and GLP-1 compound for use according to any one of the preceding claims, wherein the modulator of CD3 is a parenteral medicament.

15. The modulator of CD3 and GLP-1 compound for use according to any one of the preceding claims, wherein the modulator of CD3 and the GLP-1 compound are administered in amounts and for a sufficient time to produce a synergistic effect.

## Patentansprüche

1. Modulator von CD₃ und eine gluconartige Peptid-1(GLP-1)-Verbindung zur Verwendung bei der Vorbeugung und Intervention von Diabetes Typ 1 und latenter Autoimmundiabetes beim Erwachsenen (Latent Autoimmune Diabetes in the Adult; LADA) zur Verabreichung einem dies benötigenden Patienten, wobei der Modulator ein CD₃-Antikörper oder F(ab')2-Fragment davon ist.

2. Modulator von CD₃ und GLP-1-Verbindung zur Verwendung nach Anspruch 1, wobei der Modulator von CD₃ ausgewählt ist aus der Gruppe, bestehend aus Anti-CD₃ mAb OKT₃ oder einem F(ab')2-Fragment davon, Anti-CD₃ mAb hOKT_{3γ}(Ala-Ala) oder einem F(ab')₂-Fragment davon oder Anti-CD₃ mAb 145 2C11 oder einem F(ab')₂-Fragment davon.

3. Modulator von CD₃ und GLP-1-Verbindung zur Verwendung nach einem der vorangehenden Ansprüche, wobei die GLP-1-Verbindung ausgewählt ist aus der Gruppe, bestehend aus GLP-1(7-36)amid, GLP-1(7-37), einem Analogon davon und einem Derivat von einem des Vorstehenden.

4. Modulator von CD₃ und GLP-1-Verbindung zur Verwendung nach einem der Ansprüche 1-3, wobei die GLP-1-Verbindung ein Derivat von GLP-1(7-36)amid, GLP-1(7-37) oder ein Analogon von einem des Vorstehenden ist, das einen lipophilen Substituenten umfasst.

5. Modulator von CD₃ und GLP-1-Verbindung zur Verwendung nach einem der Ansprüche 1-4, wobei die GLP-1-Verbindung Arg³⁴, Lys²⁶(N^{ε}-(γ-Glu(N^{α}-hexadecanoyl)))-GLP-1(7-37) ist.

6. Modulator von CD₃ und GLP-1-Verbindung zur Verwendung nach einem der Ansprüche 1-4, wobei die GLP-1-Verbindung ausgewählt ist aus der Gruppe, bestehend aus Gly⁸-GLP-1(7-36)amid, Gly⁸-GLP-1(7-37), Val⁸-GLP-1(7-36)amid, Val⁸-GLP-1(7-37), val⁸Asp²²-GLP-1(7-36)amid, val⁸Asp²²-GLP-1(7-37), val⁸Glu²²-GLP-1(7-36)amid, val⁸Glu²²-GLP-1(7-37), val⁸Lys²²-GLP-1(7-36)amid, val⁸Lys²²-GLP-1(7-37), Val⁸Arg²²-GLP-1(7-36)amid, Val⁸Arg²²⁻GLP-1(7-37), val⁸His²²-GLP-1(7-36)amid, val⁸His²²-GLP-1(7-37), Analoga davon und Derivaten davon.

7. Modulator von CD₃ und GLP-1-Verbindung zur Verwendung nach einem der Ansprüche 1-2, wobei die GLP-1-Verbindung ein stabiles GLP-1-Analogon/Derivat ist.

8. Modulator von CD₃ und GLP-1-Verbindung zur Verwendung nach einem der Ansprüche 1-2, wobei die GLP-1-Verbindung Exendin-4 oder ein Analogon davon oder ein Derivat von einem des Vorstehenden ist.

9. Modulator von CD₃ und GLP-1-Verbindung zur Verwendung nach einem der Ansprüche 1-2, wobei die GLP-1-Verbindung ein stabiles Exendin-4-Analogon/Derivat ist.

10. Modulator von CD₃ und GLP-1-Verbindung zur Verwendung nach einem der vorangehenden Ansprüche, wobei die GLP-1-Verbindung parenteral oder durch Injektion zu verabreichen ist.

11. Modulator von CD₃ und GLP-1-Verbindung zur Verwendung nach Anspruch 10, wobei die Dosierung der GLP-1-Verbindung etwa 0,5 µg/kg/Tag bis etwa 20 µg/kg/Tag wie von etwa 0,1 µg/kg/Tag bis etwa 2 µg/kg/Tag beträgt.

12. Modulator von CD₃ und GLP-1-Verbindung zur Verwendung nach einem der vorangehenden Ansprüche, wobei der Modulator von CD₃ in einer Verordnung verabreicht wird, die zusätzlich die Verabreichung einer GLP-1-Verbindung umfasst.

13. Modulator von CD₃ und GLP-1-Verbindung zur Verwendung nach einem der vorangehenden Ansprüche, wobei der Modulator von CD₃ und die GLP-1-Verbindung gemeinsam verabreicht werden.

14. Modulator von CD₃ und GLP-1-Verbindung zur Verwendung nach einem der vorangehenden Ansprüche, wobei der Modulator von CD₃ ein parenterales Medikament ist.

15. Modulator von CD₃ und GLP-1-Verbindung zur Verwendung nach einem der vorangehenden Ansprüche, wobei der Modulator von CD₃ und die GLP-1-Verbindung in Mengen und für eine ausreichende Zeitdauer zum Erzeugen einer synergistischen Wirkung verabreicht werden.

## Revendications

1. Modulateur du CD3 et composé du glucagon-like peptide-1 (GLP-1) pour utilisation dans la prévention et l'intervention du diabète de type 1 et du diabète auto-immun latent chez l'adulte (LADA) pour administration à un patient qui en a besoin, ledit modulateur étant un anticorps anti-CD3 ou un fragment F(ab')₂ de ce dernier.

2. Modulateur du CD3 et composé du GLP-1 pour utilisation selon la revendication 1, le modulateur du CD3 étant choisi dans le groupe consistant en l'anticorps monoclonal anti-CD3 OKT3 ou le fragment F(ab')₂ de ce dernier, l'anticorps monoclonal anti-CD3 hOKT3γl(Ala-Ala) ou le fragment F(ab')₂ de ce dernier, ou l'anticorps monoclonal anti-CD3 145 2C11, ou le fragment F(ab')₂ de ce dernier.

3. Modulateur du CD3 et composé du GLP-1 pour utilisation selon l'une quelconque des revendications précédentes, le composé GLP-1 étant choisi dans le groupe consistant en le GLP-1 (7-36) -amide, le GLP-1 (7-37), un analogue de ceux-ci, et un dérivé de l'un quelconque des composés précédents.

4. Modulateur du CD3 et composé du GLP-1 pour utilisation selon l'une quelconque des revendications 1-3, le composé GLP-1 étant un dérivé du GLP-1(7-36)-amide, le GLP-1(7-37) ou un analogue de l'un quelconque des composés ci-dessus, qui comprend un substituant lipophile.

5. Modulateur du CD3 et composé du GLP-1 pour utilisation selon l'une quelconque des revendications 1-4, le composé GLP-1 étant Arg³⁴, Lys²⁶ (N^{ε}-(y-Glu (N^{α}-hexadécanoyle)))-GLP-1(7-37).

6. Modulateur du CD3 et composé du GLP-1 pour utilisation selon l'une quelconque des revendications 1-4, le composé GLP-1 étant choisi dans le groupe consistant en Gly⁸-GLP-1 (7-36) -amide, Gly⁸-GLP-1(7-37), Val⁸-GLP-1 (7-36) -amide, Val⁸-GLP-1-37), Val⁸Asp²²-GLP-1(7-36)-amide, Val⁸Asp²²-GLP-1 (7-37) , Val⁸Glu²²-GLP-1 (7-36)-amide, Val⁸Glu²²-GLP-1 (7-37) , Val⁸ Lys²²-GLP-1(7-36)-amide, Val⁸Lys²²-GLP-1(7-37), Val⁸ Ar g²²-GLP-1(7-36)-amide, Val⁸Arg²²-GLP-1 (7-37) , Val⁸His²²-GLP-1(7-36)-amide, Val⁸His²²-GLP-1(7-37), les analogues de ceux-ci et les dérivés de ceux-ci.

7. Modulateur du CD3 et composé du GLP-1 pour utilisation selon l'une quelconque des revendications 1-2, le composé GLP-1 étant un analogue/dérivé stable du GLP-1.

8. Modulateur du CD3 et composé du GLP-1 pour utilisation selon l'une quelconque des revendications 1-2, le composé du GLP-1 étant l'exendine-4 ou un analogue de cette dernière ou un dérivé de l'un quelconque des composés précédents.

9. Modulateur du CD3 et composé du GLP-1 pour utilisation selon l'une quelconque des revendications 1-2, le composé du GLP-1 étant un analogue/dérivé stable de l'exendine-4.

10. Modulateur du CD3 et composé du GLP-1 pour utilisation selon l'une quelconque des revendications précédentes, le composé du GLP-1 devant être administré par voie parentérale ou par injection.

11. Modulateur du CD3 et composé du GLP-1 pour utilisation selon la revendication 10, la posologie du composé du GLP-1 étant d'environ 0,5 µg/kg/jour à environ 20 µg/kg/jour, notamment d'environ 0,1 µg/kg/jour à environ 2 µg/kg/jour.

12. Modulateur du CD3 et composé du GLP-1 pour utilisation selon l'une quelconque des revendications précédentes, le modulateur du CD3 étant administré selon un schéma posologique qui comprend en outre l'administration d'un composé du GLP-1.

13. Modulateur du CD3 et composé du GLP-1 pour utilisation selon l'une quelconque des revendications précédentes, le modulateur du CD3 et le composé du GLP-1 étant co-administrés.

14. Modulateur du CD3 et composé du GLP-1 pour utilisation selon l'une quelconque des revendications précédentes, le modulateur du CD3 étant un médicament parentéral.

15. Modulateur du CD3 et composé du GLP-1 pour utilisation selon l'une quelconque des revendications précédentes, le modulateur du CD3 et le composé du GLP-1 étant administrés en des quantités et pendant un temps suffisant pour produire un effet synergique.
